# EUROPEAN PATENT APPLICATION

(11) **EP 3 791 850 A1**
(43) Date of publication of application: **17.03.2021**
(21) Application number: 19800556.3
(22) Date of filing: 10.05.2019
(51) Int. Cl.: A61J 1/20, A61M 5/162

(54) **MEDICAL HOLLOW NEEDLE AND METHOD FOR USING THE SAME**

(30) Priority: 11.05.2018 JP 2018092088
(71) Applicant: TERUMO Kabushiki Kaisha, Tokyo 151-0072 (JP)
(72) Inventor: MAEKAWA, Minami, Ashigarakami-gun, Kanagawa 259-0151 (JP); ITO, Eriko, Ashigarakami-gun, Kanagawa 259-0151 (JP); IWASE, Yoichiro, Ashigarakami-gun, Kanagawa 259-0151 (JP); TAKAKI, Toshiaki, Nakakoma-gun, Yamanashi 409-3853 (JP)
(74) Representative: TBK
(86) International application number: PCT/JP2019/018662
(87) International publication number: WO 2019/216397

(57) **Abstract**

In a hollow medical needle (10) including: a hub part (12) having an inner cavity connectable to a syringe (80); and a puncture part (14) extending out toward an axial distal-end side of the hub part (12), by forming, at a distal end of the hub part (12), a puncture index part (36) that includes a step perpendicular to an axial direction and is visible when abutting against a rubber stopper (94) when the rubber stopper (94) of a vial container (90) is punctured by the puncture part (14), a position of an insertion terminal end (38) substantially coincident with an outer surface (94a) of the rubber stopper (94) is set at a fixed position when the puncture part (14) is punctured into the rubber stopper (94).

## Description

### Technical Field

The present invention relates to a hollow medical needle and a usage method thereof.

### Background Art

Conventionally, various hollow medical needles have been proposed. For example, Re-publication of PCT International Publication No. 2016/153003 discloses a hollow medical needle made of resin. The hollow medical needle has a solid needle tip at a distal end, and a groove portion extending toward the needle tip is formed on an outer surface. At a proximal end portion of the groove portion, a side hole is provided. The side hole communicates with an inside of a hub through a communication passage that serves as a flow path of a chemical agent, and the chemical agent can be suctioned or discharged through the side hole, by operating a syringe connected to the hub.

### Summary of Invention

In a conventional hollow medical needle, it is necessary to adjust a position of a needle hole to a position lower than a liquid surface in order to suction with a small amount of residual liquid, when puncturing a medical container such as a vial container to collect a chemical agent into a syringe. However, if the hollow medical needle is excessively pulled to move the needle hole near the entrance of the medical container, a needle tip may accidentally come off from a rubber stopper of the medical container, causing a problem such as an occurrence of liquid leakage from the medical container. Therefore, in suctioning a chemical agent from a medical container with the conventional hollow medical needle, careful work has been required to adjust the position of the needle tip.

Therefore, an object of the present invention is to provide a hollow medical needle capable of easily suctioning a chemical agent from a medical container with a small amount of residual liquid, and a usage method thereof.

One aspect of the present invention is a hollow medical needle including: a hub part having an inner cavity including a female luer part to which a male luer part can be connected; a puncture part that can be punctured into a rubber stopper of a medical container, has a proximal portion having an outer diameter smaller than an outer diameter of the hub part and extending in a distal-end direction from a distal end of the hub part, and has a needle tip portion extending from a distal end of the proximal portion and having a tapered shape in which an outer diameter gradually decreases toward a distal end; and a center axis extending from a proximal end of the hub part to a distal end of the puncture part. The proximal portion has a communication passage extending along the center axis inside the proximal portion and communicating with the inner cavity of the hub part, a side hole extending from the communication passage to an outer peripheral surface of the proximal portion, and an outer diameter that is substantially uniform at least from a proximal end of the side hole to the distal end of the proximal portion. The needle tip portion has a needle tip that is solid, at the distal end. The puncture part has, on an outer peripheral surface of the puncture part, a groove portion extending from a proximal portion of the needle tip of the needle tip portion to a distal end of the side hole. The hub part has, at the distal end of the hub part, a puncture index part extending from an outer peripheral surface of the hub part toward an outer peripheral surface of the proximal portion so as to form a step surface substantially perpendicular to the center axis between the distal end of the hub part and a proximal end of the proximal portion. The proximal portion has, near the puncture index part, an insertion terminal end that is substantially coincident with an outer surface of the rubber stopper when the rubber stopper of the medical container is punctured by the puncture part. At a proximal end portion of the proximal portion, a distance L1 from the insertion terminal end to the proximal end of the side hole is 1.0 to 6.0 mm, and the puncture index part is visible from a side substantially perpendicular to the center axis, when the puncture part is punctured into the rubber stopper of the medical container.

According to the hollow medical needle of the aspect described above, the puncture index part serving as a measure of a puncture length is visible from the side. This allows the puncture part to be inserted into the rubber stopper under a certain condition, by a user visually observing and pushing the puncture index part up to the vicinity of the outer surface of the rubber stopper, when the puncture part is punctured into the rubber stopper of the medical container. As a result, a position of the insertion terminal end of the puncture part is set at a fixed position near the proximal end of the proximal portion. Furthermore, the distance L1 from the insertion terminal end to the proximal end of the side hole has been set to 1.0 mm to 6.0 mm. This enables reliable placement of the groove portion of the puncture part inside the medical container, and placement of the side hole of the puncture part on an inner side of the medical container from the outer surface of the rubber stopper, which allows a chemical agent solution to be easily suctioned from the medical container with a small amount of residual liquid, without being bothered by fine adjustment of the position of the puncture part.

### Brief Description of Drawings

Fig. 1A is a plan view of a hollow medical needle according to a first embodiment of the present invention, and Fig. 1B is a cross-sectional view taken along line IB-IB of Fig. 1A.
Fig. 2 is a plan view showing a state where the hollow medical needle of Fig. 1A is connected to a syringe.
Fig. 3 is a cross-sectional view showing a state where the hollow medical needle of Fig. 1A is punctured into a thin rubber stopper of a vial container (a medical container).
Fig. 4 is a cross-sectional view showing a state where the hollow medical needle of Fig. 1A is punctured into a thick rubber stopper of a vial container.
Fig. 5 is a plan view of a hollow medical needle according to a second embodiment of the present invention.
Fig. 6 is a cross-sectional view of a state where the hollow medical needle of Fig. 5 is pushed into a rubber stopper of a vial container.
Fig. 7 is a cross-sectional view showing a state where the rubber stopper of Fig. 6 is elastically returned.

### Description of Embodiments

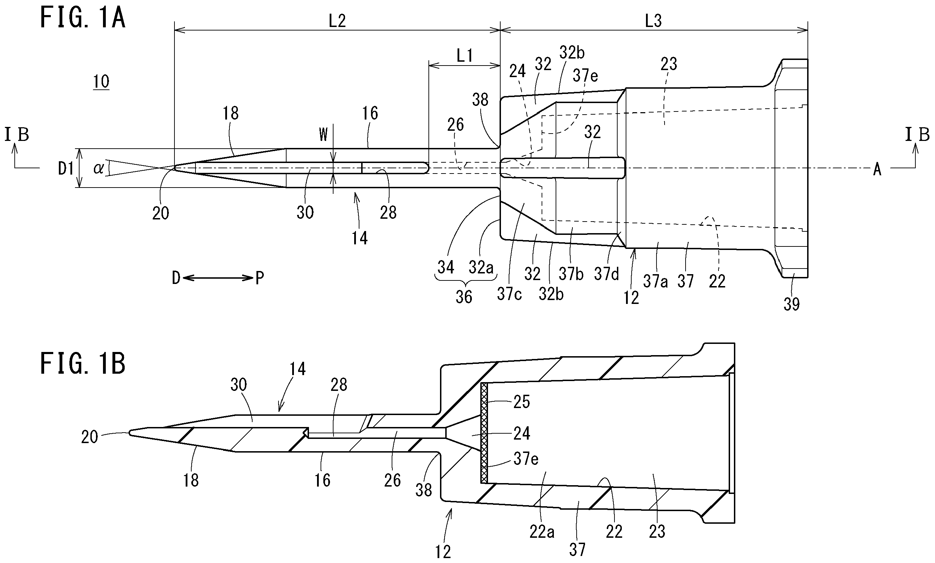

Hereinafter, preferred embodiments of the present invention will be described in detail with reference to the accompanying drawings. Note that the dimensional ratios in the drawings may be exaggerated and different from the actual ratios for convenience of description. Further, in the following description, in each member included in the hollow medical needle, an end portion on a needle tip side is referred to as a "distal end" ("D" in Fig. 1A), and an end portion in an opposite direction is referred to as a "proximal end" ("P" in Fig. 1A).

### First Embodiment

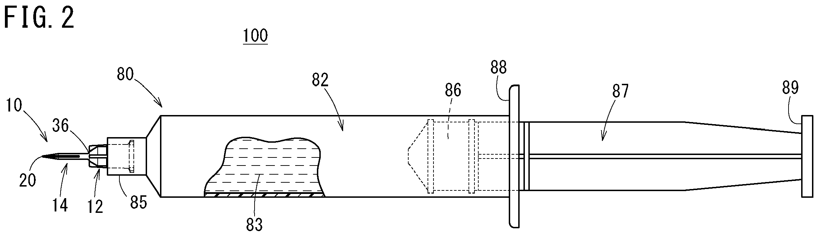

As shown in Fig. 1A, a hollow medical needle 10 according to the present embodiment includes a hub part 12, and a puncture part 14 that has a diameter smaller than that of the hub part 12 and extends out toward the distal-end side of the hub part 12. The hub part 12 is a portion to be mounted to a distal end of a syringe 80 as shown in Fig. 2. By mounting the hollow medical needle 10 on the distal end of the syringe 80, a transfer device 100 is formed.

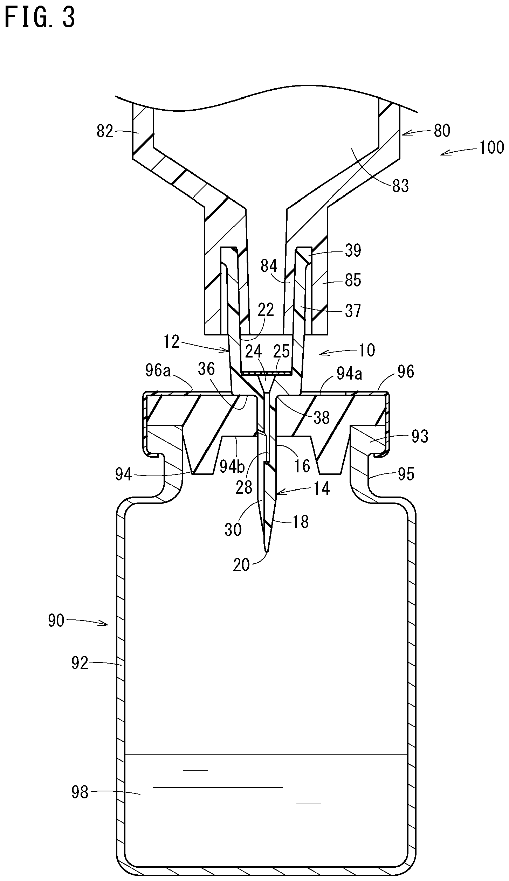

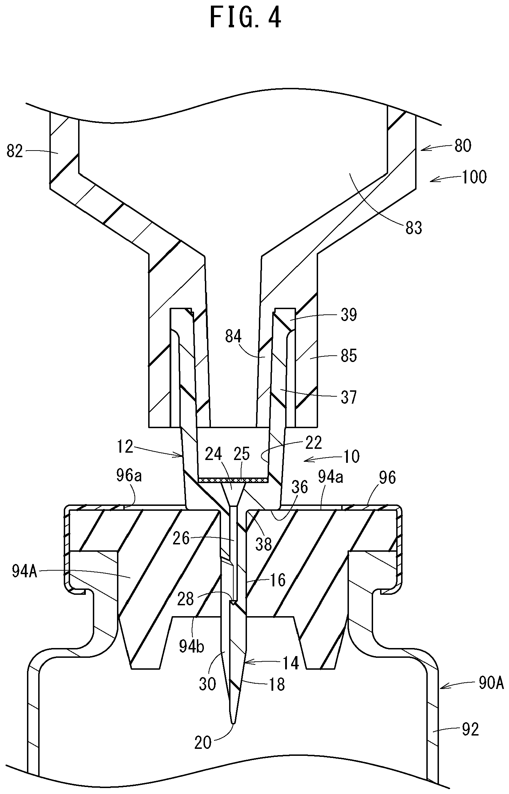

As shown in Figs. 3 and 4, the transfer device 100 including the hollow medical needle 10 is used for suctioning a chemical agent (a chemical agent solution) from an inside of a vial container 90, 90A by being punctured into a rubber stopper 94, 94A of the vial container 90, 90A. The hollow medical needle 10 is configured to be able to suction a chemical agent solution 98 in the vial container 90, 90A at that time with a small amount of residual liquid, without fine positional adjustment of the puncture part 14. Note that, in a case where the chemical agent in the vial container 90 is solid, the transfer device 100 including the hollow medical needle 10 is also used to inject a solution in the syringe 80 into the vial container 90, 90A, as described later. Hereinafter, details of the hollow medical needle 10 will be further described.

As shown in Fig. 1A, the hub part 12 of the hollow medical needle 10 includes a hub body 37, a protruding portion 39, a rib 32, and an inner cavity 23.

The hub body 37 is formed in a cylindrical shape extending in a direction (an axial direction) of a center axis A of the hollow medical needle 10. The hub body 37 includes a proximal end portion 37a formed on a proximal end side. The proximal end portion 37a is formed with substantially the same outer diameter in the axial direction. At a distal end of the proximal end portion 37a, an intermediate portion 37b is formed via a step portion 37d. The intermediate portion 37b is a portion formed in a cylindrical shape and having an outer diameter smaller than that of the proximal end portion 37a. Further, at a distal end of the intermediate portion 37b, there is formed a narrowed portion 37c formed in a tapered shape and having an outer diameter gradually decreasing toward a distal end. At the distal end of the narrowed portion 37c, a distal-end surface 34 that forms a part of a puncture index part 36 to be described later is formed. The distal-end surface 34 has a surface perpendicular to the center axis A.

The protruding portion 39 is formed at a proximal end of the proximal end portion 37a. A plurality of protruding portions 39 may be provided, and, in that case, may be provided at even intervals in a circumferential direction of the proximal end portion 37a. This protruding portion 39 is configured to be engageable with a thread groove on the syringe 80 side, when the hollow medical needle 10 is mounted to the distal end of the syringe 80.

The rib 32 is formed to extend out radially outward from the intermediate portion 37b and the narrowed portion 37c. A plurality of ribs 32 may be provided at predetermined intervals in a circumferential direction of the intermediate portion 37b and the narrowed portion 37c. The rib 32 also functions as a slip stopper that engages with a packing container (not shown), to prevent corotation of the hollow medical needle 10 when the hollow medical needle 10 is screwed and mounted into the syringe 80. Further, an outer side surface 32b of the rib 32 may be formed in a tapered shape that gradually approaches the axial direction toward the distal-end side. The outer side surface 32b is formed in the step portion 37d at the same position as an outer peripheral surface of the proximal end portion 37a. Further, at a distal end of the rib 32, a distal-end surface 32a perpendicular to the axial direction is formed. This distal-end surface 32a of the rib 32 is included in the same surface (a step surface) as the distal-end surface 34 of the narrowed portion 37c. This step surface formed by the distal-end surface 34 of this narrowed portion 37c and the distal-end surface 32a of the rib 32 serves as the puncture index part 36.

As shown in Fig. 1B, the inner cavity 23 is a cavity portion formed in a tapered shape in an inside of the hub body 37, and includes a female luer part 22, a distal-end inner cavity 22a, and a through hole 24. The female luer part 22 is open at the proximal end side and can accommodate a male luer part 84 (see Fig. 3) of the syringe 80. The female luer part 22 extends up to a distal-end wall 37e of the narrowed portion 37c. The female luer part 22 is formed to have an inner surface shape that can be liquid-tightly in close contact with the male luer part 84 on the syringe 80 side. Specifically, for example, an inner surface of the female luer part 22 has a taper angle inclined by 6% with respect to the axial direction, to allow connection of a normal standard medical female connector (the male luer part 84) having a 6% taper. The normal standard medical female connector is a connector that meets ISO 80369-7:2016, "Small-bore connectors for liquids and gases in healthcare applications -- Part 7: Connectors for intravascular or hypodermic applications". Note that the female luer part 22 does not need to extend up to the distal-end wall 37e of the narrowed portion 37c.

At a distal end portion of the distal-end inner cavity 22a, a filter 25 is provided. The filter 25 is arranged so as to separate a chemical-agent storage part 83 of the syringe 80 from a communication passage 26. As the filter 25, for example, one having a hole diameter of about 5 µm can be used. This filter 25 removes foreign matter mixed by coring or the like in a chemical agent solution. Note that the filter 25 need not be provided in the distal-end inner cavity 22a.

The through hole 24 is a hole that axially penetrates the distal-end wall 37e of the narrowed portion 37c, and has a distal end communicating with the communication passage 26 of the puncture part 14. Although not particularly limited, the through hole 24 may be formed in a tapered shape in which an inner diameter decreases from a proximal end to the distal end.

An axial length L3 from the proximal end to the distal end of the hub part 12 can be set to 7.5 mm to 17.3 mm.

As shown in Fig. 1A, the puncture part 14 includes a proximal portion 16, a needle tip portion 18, a needle tip 20, a side hole 28, and a groove portion 30. The proximal portion 16 extends from the puncture index part 36 at the distal end of the hub part 12 toward an axial distal-end side. The proximal portion 16 is formed in a cylindrical shape having a uniform outer diameter in the axial direction. An outer diameter D1 of the puncture part 14 (the proximal portion 16) can be set to 0.7 to 4.0 mm, for example. By setting the outer diameter D1 to 0.7 mm or more, it is possible to secure strength required for puncturing the rubber stopper 94 of the vial container 90 (see Fig. 3) or a mixed injection port on an intravenous line (a transfusion line) connected to a vein of a living body. Further, by setting the outer diameter D1 to be 4.0 mm or less, it becomes also possible to puncture, for example, a mixed injection port of an infusion bag or the like having a small diameter, without any trouble.

The needle tip portion 18 extends out from the proximal portion 16 toward the axial distal-end side, and is formed in a tapered shape in which an outer diameter gradually decreases toward a distal end. At the distal end of the needle tip portion 18, the needle tip 20 is formed. The needle tip portion 18 and the needle tip 20 are formed to be solid. A taper angle α of the needle tip portion 18 is preferably set to 10 to 30 degrees so that a port of an infusion container made of a hard material can be reliably punctured.

As shown in Fig. 1B, in the proximal portion 16, there is formed the communication passage 26 that serves as a flow path for the chemical agent in the proximal portion 16. The communication passage 26 is formed inside the proximal portion 16 along an axial center of the hub part 12 and the puncture part 14. A proximal end of the communication passage 26 communicates with the through hole 24 of the hub part 12. A distal end of the communication passage 26 communicates with the side hole 28.

As shown in Fig. 1A, the side hole 28 is formed to be elongated in the axial direction and opens on an outer peripheral surface of the proximal portion 16. As shown in Fig. 3, when the hollow medical needle 10 is punctured into the vial container 90 to suction the chemical agent, the chemical agent can be suctioned in a range up to a position of a proximal end of the side hole 28. Therefore, if the proximal end of the side hole 28 enters inside the vial container 90 to be too far from an inner surface 94b of the rubber stopper 94 of the vial container 90, residual liquid will be generated in the vial container 90. Note that, in the present embodiment, the side hole 28 is formed to be elongated in the axial direction, but may be circular or square in a side view (plan view of Fig. 1A).

Therefore, the position of the proximal end of the side hole 28 is preferably set in a range that does not excessively enter inside the rubber stopper 94, when the puncture part 14 is punctured into the rubber stopper 94 of the vial container 90. Specifically, when the puncture part 14 is punctured into the rubber stopper 94, it is preferable to set an axial distance L1 to the proximal end of the side hole 28 from an insertion terminal end 38 coincident with an outer surface 94a of the rubber stopper 94 of the puncture part 14, to 1.0 to 6.0 mm. Note that, since the insertion terminal end 38 of the hollow medical needle 10 of the present embodiment is near the puncture index part 36, a starting point of the distance L1 may be the puncture index part 36.

An axial length of the side hole 28 is preferably a length with which the side hole 28 is located near the inner surface 94b of the rubber stopper 94 and does not open to the outside of the vial container 90, when the puncture part 14 is punctured until the puncture index part 36 abuts against the rubber stopper 94 of the vial container 90. Specifically, for example, the axial length of the side hole 28 can be set to 0.1 to 25 mm.

From the distal end of the side hole 28, the groove portion 30 extends toward an axial distal end. The groove portion 30 is formed from the outer peripheral surface of the proximal portion 16 to the needle tip portion 18. A groove width W (a width in a direction perpendicular to the center axis A) of the groove portion 30 is preferably set to a value smaller than the outer diameter D1 of the puncture part 14 (the proximal portion 16) by 0.6 mm or more. Specifically, the groove width W of the groove portion 30 can be set to 0.1 to 3.4 mm. Further, the groove portion 30 has a groove depth of 0.1 to 1.9 mm in a radial direction. Note that the groove depth of the groove portion 30 is preferably smaller than the outer diameter of the proximal portion 16 by 0.6 mm or more. The depth of the groove portion 30 is preferably set to a depth that is not blocked by the rubber stopper 94 when the rubber stopper 94 of the vial container 90 is punctured.

Note that a plurality of the above-described side holes 28 and groove portions 30 may be formed on the outer peripheral surfaces of the proximal portion 16 and the needle tip portion 18 with a predetermined distance in the circumferential direction. Further, an axial length L2 of the puncture part 14 can be set to, for example, 5 to 34 mm.

The hollow medical needle 10 described above may be made of either metal or resin, but is preferably made of resin for ease of shaping.

As shown in Fig. 2, the hollow medical needle 10 is mounted to the distal end of the syringe 80. The syringe 80 is, for example, a pre-filled syringe, and includes an outer cylinder 82, a connecting part 85 provided at a distal end portion of the outer cylinder 82, a gasket 86 slidably accommodated inside the outer cylinder 82, and a pusher 87 mounted to the gasket 86. Between the gasket 86 and the outer cylinder 82, the chemical-agent storage part 83 is formed, and the chemical agent is stored in the chemical-agent storage part 83. Further, at a rear end portion of the outer cylinder 82, a flange portion 88 is formed.

The outer cylinder 82 is a member formed of a transparent or semi-transparent material in a cylindrical shape. As shown in Fig. 3, the connecting part 85 is formed at the distal end portion of the outer cylinder 82. At the connecting part 85, a luer taper nozzle (hereinafter, a male luer part) 84 is formed. The male luer part 84 may have a shape that can be connected to a normal standard medical female connector. The male luer part 84 is inserted into the female luer part 22 of the hollow medical needle 10 to be liquid-tightly connected.

As shown in Fig. 2, the gasket 86 has an outer diameter that is substantially the same as an inner diameter of the outer cylinder 82, and slides in liquid-tight contact with an inner wall of the outer cylinder 82. To a rear end portion of the gasket 86, the pusher 87 is connected.

The pusher 87 includes a pressing part 89 at a rear end portion, and transmits a force from the pressing part 89 to the gasket 86. The pusher 87 may be configured to be attached to the gasket 86 at the time of use.

Next, the vial container (the medical container) 90 to be punctured by the hollow medical needle 10 will be described. As shown in Fig. 3, the vial container 90 internally accommodates a chemical agent. This chemical agent is powdery or granular solid or liquid. In a case where the chemical agent is solid, it is dissolved by a solution injected from the syringe 80, to be prepared as the chemical agent solution 98. Thereafter, the chemical agent solution 98 is transferred from the vial container 90 to the syringe 80 via the hollow medical needle 10, to be used. In a case where the chemical agent is a liquid chemical agent solution in advance, it is mixed with a diluting liquid (e.g., distilled water) as needed to be diluted to a desired concentration, and then transferred from the vial container 90 to the syringe 80 via the hollow medical needle 10, to be used.

The vial container 90 includes a hard container body 92, the rubber stopper 94 that is made of an elastic material and hermetically seals a mouth portion 93 of the container body 92, and a cap 96 that covers the mouth portion 93 of the container body 92 and an outer circumference of the rubber stopper 94. The container body 92 is made of, for example, a material such as various kinds of glass or various kinds of resin, and is formed to be transparent or semi-transparent in order to secure internal visibility.

The container body 92 has the mouth portion 93 included in an upper end portion, and a neck portion 95 formed below the mouth portion 93. An outer diameter of the neck portion 95 is smaller than an outer diameter of the mouth portion 93.

The cap 96 is made of a soft metal thin plate such as aluminum, for example, and has an opening 96a formed on an outer surface thereof, and the outer surface 94a of the rubber stopper 94 is exposed from the opening 96a. The cap 96 covers an outer peripheral surface of the rubber stopper 94, an outer surface edge portion of the rubber stopper 94, an outer peripheral surface of the mouth portion 93, and a lower end edge portion of the mouth portion 93.

The hollow medical needle 10 of the present embodiment is punctured into the rubber stopper 94 of the vial container 90 while being mounted to the syringe 80. Hereinafter, an operation of the hollow medical needle 10 will be described.

As shown in Fig. 2, by inserting the male luer part 84 (see Fig. 3) of the syringe 80 into the hub part 12 of the hollow medical needle 10, the hollow medical needle 10 is mounted to the syringe 80, and the transfer device 100 is assembled.

Next, as shown in Fig. 3, the vial container 90 is prepared, and the rubber stopper 94 of the vial container 90 is punctured by the puncture part 14 of the hollow medical needle 10. At that time, as shown in the figure, the puncture part 14 is pushed into the rubber stopper 94 such that an end surface included in the puncture index part 36 abuts against the outer surface 94a of the rubber stopper 94. This causes the puncture part 14 to be inserted into the rubber stopper 94 up to the vicinity of a proximal end of the proximal portion 16. A part of the groove portion 30 and the side hole 28 of the puncture part 14 is exposed inside the inner surface 94b of the rubber stopper 94. At that time, the proximal end of the side hole 28 is located between the outer surface 94a and the inner surface 94b of the rubber stopper 94 and near the inner surface 94b. The chemical-agent storage part 83 of the syringe 80 and an inside of the vial container 90 communicate with each other via the side hole 28 of the hollow medical needle 10. Note that, in puncturing the rubber stopper 94 with the puncture part 14, while the rubber stopper 94 is elastically deformed toward an inner side of the vial container (the medical container) 90 by the proximal portion 16 of the puncture part 14 punctured into the rubber stopper 94, the puncture part 14 is inserted into the rubber stopper 94 until the puncture index part 36 becomes coincident with an outer surface position where the outer surface 94a of the rubber stopper 94 before elastic deformation has been located, or is located on an inner side of the vial container 90 from this outer surface position. Thereafter, by causing the rubber stopper 94 to be elastically returned toward the outside of the vial container 90, the insertion terminal end 38 of the proximal portion 16 of the puncture part 14 is made substantially coincident with the outer surface 94a of the rubber stopper 94. This allows the groove portion 30 of the puncture part 14 to be exposed inside the inner surface 94b of the rubber stopper 94, and the proximal end of the side hole 28 to be located between the outer surface 94a and the inner surface 94b of the rubber stopper 94.

Thereafter, the chemical agent solution 98 is prepared in the vial container 90, and then the chemical agent solution 98 in the vial container 90 is suctioned. For suctioning the chemical agent solution 98, the top and bottom of the vial container 90 are reversed from the state of Fig. 3, the chemical agent solution 98 is collected on the rubber stopper 94 side, and the pusher 87 (see Fig. 2) of the syringe 80 is pulled. The chemical agent solution 98 in the vial container 90 is transferred to the syringe 80 through the side hole 28 of the hollow medical needle 10.

Next, an example will be described in which the hollow medical needle 10 is punctured into a vial container 90A having a thick rubber stopper 94A.

Also in this case, as shown in Fig. 4, the puncture part 14 of the hollow medical needle 10 is pushed into the rubber stopper 94A until the puncture index part 36 of the hollow medical needle 10 abuts against an outer surface 94a of the rubber stopper 94A. This causes the puncture part 14 to be inserted into the rubber stopper 94A up to the vicinity of the proximal end of the proximal portion 16. A part of the puncture part 14 near a distal end of the groove portion 30 is exposed inside an inner surface 94b of the rubber stopper 94A. At that time, the side hole 28 is located between the outer surface 94a and the inner surface 94b of the rubber stopper 94A. Inside the rubber stopper 94A, a flow path communicating with the side hole 28 is formed by the groove portion 30. Therefore, the chemical-agent storage part 83 of the syringe 80 and the inside of the vial container 90A communicate with each other via the groove portion 30 and the side hole 28 of the hollow medical needle 10. Note that, also in this case, by a puncturing method for the rubber stopper 94 with the puncture part 14 described above, the groove portion 30 of the puncture part 14 can be exposed inside the inner surface 94b of the rubber stopper 94A, and the proximal end of the side hole 28 can be located between the outer surface 94a and the inner surface 94b of the rubber stopper 94A.

Thereafter, the chemical agent solution 98 is prepared in the vial container 90A, and then the chemical agent solution 98 in the vial container 90A is suctioned. For suctioning the chemical agent solution 98, the top and bottom of the vial container 90A are reversed from the state of Fig. 4, the chemical agent solution 98 is collected on the rubber stopper 94A side, and the pusher 87 (see Fig. 2) of the syringe 80 is pulled. The chemical agent solution 98 in the vial container 90A passes through the groove portion 30 of the hollow medical needle 10 to reach the side hole 28, and is transferred to the syringe 80 via the communication passage 26 communicating with the side hole 28.

The hollow medical needle 10 described above has the following effects.

According to the hollow medical needle 10, by pushing the puncture part 14 into the rubber stopper 94 of the vial container (the medical container) 90 up to the vicinity of the outer surface 94a of the rubber stopper 94 while the user visually checks the puncture index part 36, the puncture part 14 can be pushed into the rubber stopper 94 under a certain condition. As a result, a position of the insertion terminal end 38 of the puncture part 14 is set at a fixed position near the proximal end of the proximal portion 16. Further, the distance L1 from the insertion terminal end 38 to the proximal end of the side hole 28 is 1.0 to 6.0 mm. This allows the groove portion 30 of the puncture part 14 to be reliably placed inside the vial container 90, and the side hole 28 of the puncture part 14 to be placed on the inner side of the vial container 90 from the outer surface 94a of the rubber stopper 94, and allows the chemical agent solution 98 to be easily suctioned from the vial container 90 with a small amount of residual liquid, without being bothered by fine adjustment of the position of the puncture part 14.

In the hollow medical needle 10 described above, the outer diameter D1 of the puncture part 14 may be 0.7 to 4.0 mm. This facilitates not only puncturing into the vial container 90, but also puncturing into a thin port of an infusion container.

In the hollow medical needle 10 described above, the groove width W of the groove portion 30 may be 0.1 to 3.4 mm, and may be smaller than the outer diameter D1 of the puncture part 14 by 0.6 mm or more. By setting the groove width W of the groove portion 30 to such a value, it is possible to prevent a problem that the groove portion 30 is filled with the rubber stopper 94.

In the hollow medical needle 10 described above, a groove depth of the groove portion 30 in the radial direction may be 0.1 to 1.9 mm, and may be smaller than the outer diameter D1 of the puncture part 14 (the proximal portion 16) by 0.6 mm or more. By setting the groove depth of the groove portion 30 to such a value, it is possible to prevent a problem that the groove portion 30 is filled with the rubber stopper 94.

In the hollow medical needle 10 described above, a distal-end angle of the needle tip portion 18 may be 10 to 30 degrees. Adopting such a distal-end angle allows a port for an infusion container made of a hard material to be reliably punctured.

In the hollow medical needle 10 described above, the hub part 12 may have at least one rib 32 protruding from an outer peripheral surface of the distal end portion of the hub part 12 in a direction perpendicular to the center axis. Providing such a rib 32 facilitates an operation of screwing and mounting the hollow medical needle 10 into the syringe 80.

In the hollow medical needle 10 described above, the distal-end surface 32a of the rib 32 may form at least a part of the puncture index part 36. This can simplify a structure of the puncture index part 36, by the distal-end surface 32a of the rib 32 also serving as a part of the puncture index part 36.

In the hollow medical needle 10 described above, there may be provided the filter 25 that separates the female luer part 22 of the syringe 80 from the communication passage 26, in the inner cavity 23 of the hub part 12. This enables removal of foreign matter in the chemical agent solution.

In the hollow medical needle 10 described above, when the puncture part 14 is punctured into the rubber stopper 94A of the vial container (the medical container) 90A having a thickness greater than the distance L1 between the insertion terminal end 38 and the proximal end of the side hole 28, and the insertion terminal end 38 of the proximal portion 16 is made substantially coincident with the outer surface 94a of the rubber stopper 94A, at least the distal end portion of the groove portion 30 of the puncture part 14 may be exposed inside the vial container (the medical container) 90A, the proximal end portion of the groove portion 30 and the side hole 28 may be placed between the inner surface 94b and the outer surface 94a of the rubber stopper 94A, and a bottom portion of the groove portion 30 may be separated from the rubber stopper 94A. This enables the chemical agent solution 98 to be suctioned from the vial container (the medical container) 90A with a small amount of residual liquid.

In the hollow medical needle 10 described above, the communication passage 26 may be formed on an axis of the hub part 12 and the proximal portion 16. This allows the side hole 28 and the groove portion 30 to be formed in any direction. Further, it becomes also possible to provide a plurality of side holes 28 and groove portions 30 in a circumferential direction.

In the hollow medical needle 10 described above, the inner cavity 23 of the hub part 12 may have the female luer part 22 that receives the male luer part 84 of the syringe 80, and the through hole 24 that is formed to penetrate the distal-end wall 37e of the hub part 12 and connects the female luer part 22 and the communication passage 26, and the length L3 from the distal end to the proximal end of the hub part 12 may be 7.5 mm to 17.3 mm.

### Second Embodiment

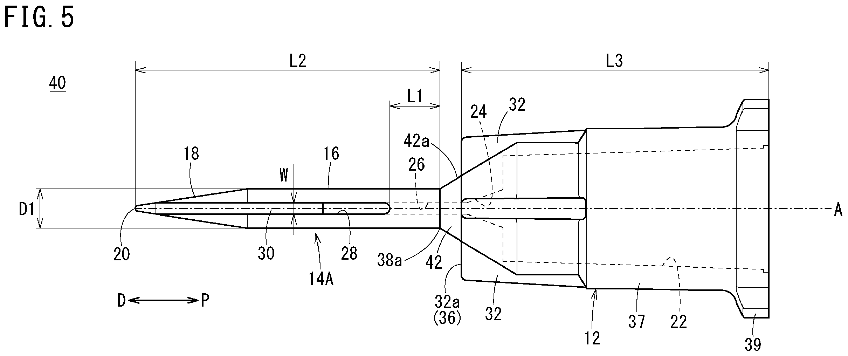

As shown in Fig. 5, a hollow medical needle 40 according to the present embodiment differs from the hollow medical needle 10 according to the first embodiment (Fig. 1A) in that a tapered skirt portion 42 is formed between a puncture index part 36 and a puncture part 14A. Hereinafter, in the hollow medical needle 40, components similar to those of the hollow medical needle 10 will be denoted by the same reference numerals, and detailed description thereof will be omitted.

The puncture part 14A is provided with the skirt portion 42 on a proximal portion 16 thereof. The skirt portion 42 extends out from a distal end of a hub part 12 toward an axial distal end. An outer diameter of a proximal end of the skirt portion 42 is formed smaller than an outer diameter of the puncture index part 36. On an outside of the proximal end of the skirt portion 42, the puncture index part 36 including a surface perpendicular to an axial direction is shown. Further, a distal end of the skirt portion 42 is connected to the proximal portion 16 of the puncture part 14A. The skirt portion 42 has a tapered outer peripheral surface 42a having an outer diameter decreasing toward the distal-end side. That is, the outer diameter of the skirt portion 42 decreases at a constant rate toward the distal-end side, and a taper angle of the skirt portion 42 is constant from a proximal end portion to a distal end portion of the skirt portion 42. An outer diameter at the distal end of the skirt portion 42 is formed to be substantially the same as an outer diameter of the proximal portion 16.

The taper angle of the skirt portion 42 (an angle formed by the outer peripheral surface 42a with a center axis A) can be set to, for example, 2 to 70 degrees. Further, an axial length of the skirt portion 42 (a length along the center axis) can be set to 2.0 to 8.0 mm.

A communication passage 26 penetrates an axial center of the skirt portion 42 in the axial direction.

In the present embodiment, the distal end of the skirt portion 42 serves as an insertion terminal end 38a of the puncture part 14A. Therefore, a proximal end of a side hole 28 is formed at a position separated from the distal end of the skirt portion 42 by L1. Also in the present embodiment, the value of L1 can be set to 1.0 to 6.0 mm.

Hereinafter, an operation of the hollow medical needle 40 of the present embodiment will be described.

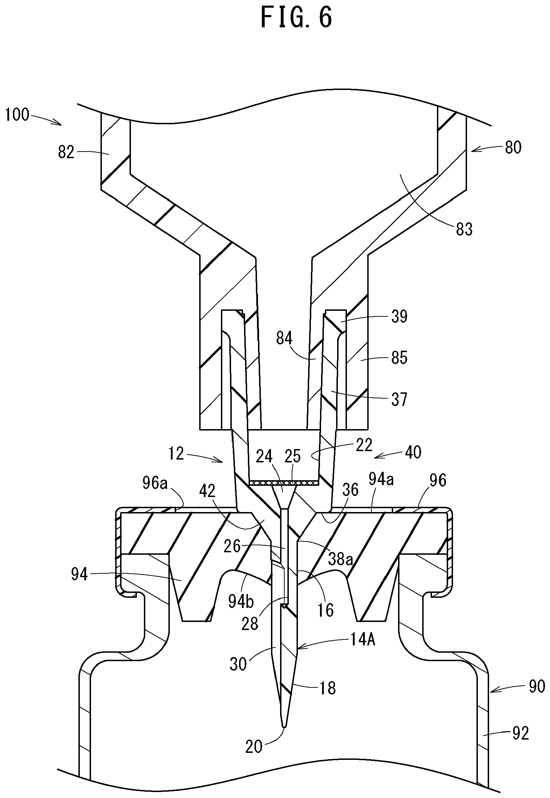

First, as shown in Fig. 6, the hub part 12 of the hollow medical needle 40 is connected to a male luer part 84 of a syringe 80, and a transfer device 100 is assembled.

Next, the puncture part 14A of the hollow medical needle 40 is punctured into a rubber stopper 94 of a vial container 90. In the present embodiment, as shown in the figure, the puncture part 14A is pushed into the rubber stopper 94 until the puncture index part 36 abuts against the rubber stopper 94. The rubber stopper 94 elastically deforms toward an inner side of the vial container 90 with the puncturing of the puncture part 14A. Then, by the skirt portion 42 coming into contact with an outer surface 94a of the rubber stopper 94, the insertion of the puncture part 14A is stopped. With a pushing force by the user causing the puncture index part 36 to abut against the outer surface 94a of the rubber stopper 94, a part of the skirt portion 42 is pushed out of the rubber stopper 94 even if being inserted inside the rubber stopper 94.

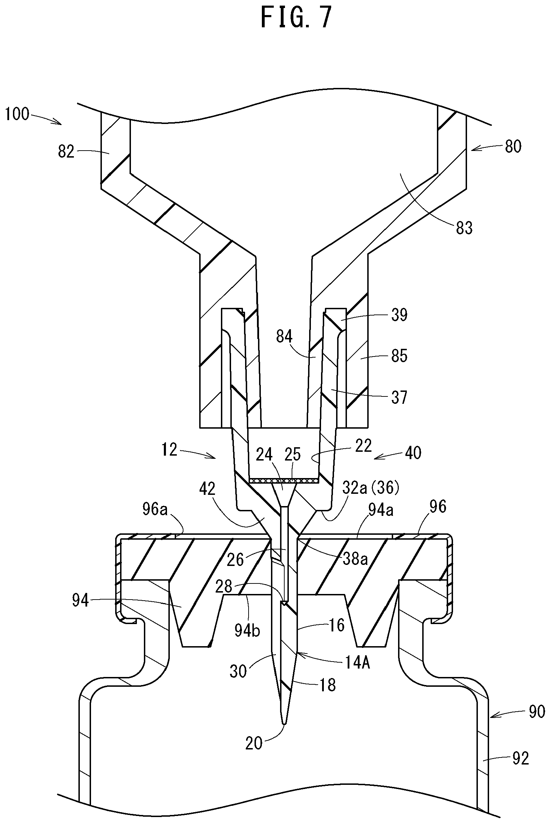

Thereafter, as shown in Fig. 7, the pushing of the hollow medical needle 40 is stopped, and the rubber stopper 94 is elastically returned. This causes the skirt portion 42 to be pushed back above the rubber stopper 94. As a result, a part the puncture part 14A up to a proximal end of the proximal portion 16 is inserted into the rubber stopper 94. That is, the distal end of the skirt portion 42 becomes the insertion terminal end 38a of the puncture part 14A.

This causes a proximal portion of the side hole 28 to be placed between the outer surface 94a and the inner surface 94b of the rubber stopper 94 as shown in the figure, at least a part of the side hole 28 and a groove portion 30 to be exposed on the inner side of the rubber stopper 94, and the chemical-agent storage part 83 and an inside of the vial container 90 to communicate with each other via the hollow medical needle 40. This enables preparation of a chemical agent solution 98 and suction of the chemical agent solution 98 from the vial container 90. Note that, also in this case, by a puncturing method similar to the puncturing method for the rubber stopper 94 with the puncture part 14 described above, the groove portion 30 of the puncture part 14A can be exposed inside the inner surface 94b of the rubber stopper 94, and the proximal end of the side hole 28 can be located between the outer surface 94a and the inner surface 94b of the rubber stopper 94.

The hollow medical needle 40 of the present embodiment has the following effects.

In a conventional hollow medical needle, even if the insertion terminal end 38a of the puncture part 14A is intended to be inserted at a fixed position, the puncture part 14A may be merely inserted apparently due to the elastic deformation of the rubber stopper 94, and the position of the insertion terminal end 38a may not be fixed. Therefore, there has been a possibility that the position of the side hole 28 is not placed at an appropriate position, and liquid leaks from the vial container 90 or residual liquid is generated in the vial container 90.

On the other hand, the hollow medical needle 40 includes the skirt portion 42 extending out from a step surface of the puncture index part 36 and formed in a tapered shape having an outer diameter gradually increasing toward the proximal end of the proximal portion 16. As a result, by puncturing such that the puncture index part 36 of the hollow medical needle 40 abuts against the rubber stopper 94 of the vial container 90, the distal end of the skirt portion 42 becomes the insertion terminal end 38a. That is, the position of the insertion terminal end 38a is fixed at the distal end of the skirt portion 42. Therefore, the side hole 28 and the groove portion 30 can always be placed at appropriate positions, and the chemical agent solution can be easily suctioned from the vial container 90 with a small amount of residual liquid, without being bothered by fine adjustment of the puncture part 14A.

In the hollow medical needle 40 described above, the taper angle of the skirt portion 42 can be set to 2 to 70 degrees. This allows the position of the insertion terminal end 38a to be made coincident with the distal end of the skirt portion 42 while preventing the distal end of the skirt portion 42 from being buried in the rubber stopper 94.

In the hollow medical needle 40 described above, an axial length of the skirt portion 42 (a length along the center axis) may be 2.0 to 8.0 mm. Adopting such a range of length allows the position of the insertion terminal end 38a to be made coincident with the distal end of the skirt portion 42 while preventing the distal end of the skirt portion 42 from being buried in the rubber stopper 94, under a pushing force that causes the puncture index part 36 to abut against the outer surface 94a of the rubber stopper 94 or to approach the vicinity thereof.

Further, the hollow medical needle 40 can be applied with a usage method including a step of connecting the hollow medical needle 40 to the syringe 80, a step of puncturing the rubber stopper 94 of the vial container 90 with the puncture part 14A, a step of pushing the puncture part 14A into the rubber stopper 94 to elastically deform the rubber stopper 94 toward an inner side of the vial container 90, and causing the puncture index part 36 to abut against the rubber stopper 94 or to come closer to the outer surface 94a thereof, and a step of causing the rubber stopper 94 to elastically return. By adopting such a usage method, the side hole 28 and the groove portion 30 can always be placed at appropriate positions, and the chemical agent solution can be easily suctioned from the vial container 90 with a small amount of residual liquid, without being bothered by fine adjustment of the puncture part 14A.

The present invention is not limited to the embodiments described above, and various modifications can be made without departing from the spirit of the present invention.

## Claims

1. A hollow medical needle comprising:
a hub part having an inner cavity including a female luer part to which a male luer part can be connected;
a puncture part that can be punctured into a rubber stopper of a medical container, wherein the puncture part has a proximal portion having an outer diameter smaller than an outer diameter of the hub part and extending in a distal-end direction from a distal end of the hub part, and has a needle tip portion extending from a distal end of the proximal portion and having a tapered shape in which an outer diameter gradually decreases toward a distal end; and
a center axis extending from a proximal end of the hub part to a distal end of the puncture part, wherein
the proximal portion has a communication passage extending along the center axis inside the proximal portion and communicating with the inner cavity of the hub part, a side hole extending from the communication passage to an outer peripheral surface of the proximal portion, and an outer diameter that is substantially uniform at least from a proximal end of the side hole to the distal end of the proximal portion,
the needle tip portion has a needle tip that is solid, at the distal end,
the puncture part has, on an outer peripheral surface of the puncture part, a groove portion extending from a proximal portion of the needle tip of the needle tip portion to a distal end of the side hole,
the hub part has, at the distal end of the hub part, a puncture index part extending from an outer peripheral surface of the hub part toward an outer peripheral surface of the proximal portion, to form a step surface substantially perpendicular to the center axis between the distal end of the hub part and a proximal end of the proximal portion,
the proximal portion has, near the puncture index part, an insertion terminal end that is substantially coincident with an outer surface of the rubber stopper when the rubber stopper of the medical container is punctured by the puncture part,
at a proximal end portion of the proximal portion,
a distance L1 from the insertion terminal end to the proximal end of the side hole is 1.0 to 6.0 mm, and
the puncture index part is visible from a side substantially perpendicular to the center axis, when the puncture part is punctured into the rubber stopper of the medical container.

2. The hollow medical needle according to claim 1, wherein
the puncture part has an outer diameter of 0.7 to 4.0 mm.

3. The hollow medical needle according to claim 2, wherein
the groove portion of the puncture part has a groove width of 0.1 to 3.4 mm in a direction perpendicular to the center axis, and
the groove width of the groove portion is smaller than the outer diameter of the puncture part by 0.6 mm or more.

4. The hollow medical needle according to claim 2, wherein
the groove portion of the puncture part has a groove depth of 0.1 to 1.9 mm in a radial direction, and
the groove depth of the groove portion is smaller than the outer diameter of the puncture part by 0.6 mm or more.

5. The hollow medical needle according to any one of claims 1 to 4, wherein
the needle tip portion has a distal-end angle of 10 to 30 degrees.

6. The hollow medical needle according to any one of claims 1 to 5, wherein
the hub part has at least one rib protruding from an outer peripheral surface of a distal end portion of the hub part in a direction perpendicular to the center axis.

7. The hollow medical needle according to claim 6, wherein
a distal-end surface of the rib forms at least a part of the puncture index part.

8. The hollow medical needle according to any one of claims 1 to 7, wherein
the hub part has a filter arranged in the inner cavity to separate the female luer part from the communication passage of the proximal portion.

9. The hollow medical needle according to any one of claims 1 to 8, wherein
the proximal portion has, at the proximal end portion of the proximal portion, a skirt portion formed in a tapered shape to allow an outer diameter of the proximal portion to gradually increase up to the proximal end of the proximal portion, and
a distal end of the skirt portion is included in the insertion terminal end.

10. The hollow medical needle according to claim 9, wherein
a taper angle of the skirt portion is 2 to 70 degrees.

11. The hollow medical needle according to claim 9 or 10, wherein
a length of the skirt portion along the center axis is 2.0 to 8.0 mm.

12. The hollow medical needle according to any one of claims 1 to 11, wherein
when the puncture part is punctured into the rubber stopper of the medical container having a thickness greater than the distance L1, and the insertion terminal end of the proximal portion is made substantially coincident with the outer surface of the rubber stopper, at least a distal end portion of the groove portion of the puncture part is exposed inside the medical container, a proximal end portion of the groove portion and the side hole of the proximal portion are placed between an inner surface and the outer surface of the rubber stopper, and a bottom portion of the proximal end portion of the groove portion is separated from the rubber stopper.

13. The hollow medical needle according to any one of claims 1 to 12, wherein
an extension axis of the communication passage is substantially coincident with the center axis.

14. The hollow medical needle according to any one of claims 1 to 13, wherein
an inner cavity of the hub part has a distal-end inner cavity extending from the female luer part in the distal-end direction, a distal-end wall provided at a distal end of the distal-end inner cavity, and a through hole penetrating the distal-end wall and connecting the distal-end inner cavity and the communication passage, and
a length L3 from the distal end to the proximal end of the hub part is 7.5 to 17.3 mm.

15. A usage method of the hollow medical needle according to any one of claims 1 to 14, the usage method comprising:
preparing the hollow medical needle;
puncturing the puncture part into the rubber stopper of the medical container;
causing the rubber stopper to elastically deform toward an inner side of the medical container by the proximal portion of the puncture part punctured into the rubber stopper, and inserting the puncture part into the rubber stopper until the puncture index part becomes coincident with an outer surface position where the outer surface of the rubber stopper before elastic deformation has been located, or is located on the inner side of the medical container from the outer surface position;
exposing at least a distal end portion of the groove portion of the puncture part inside the medical container; and
causing the rubber stopper to elastically return toward an outer side of the medical container, to make the insertion terminal end of the proximal portion to be substantially coincident with the outer surface of the rubber stopper.
